# EUROPEAN PATENT APPLICATION

(11) **EP 4 159 866 A1**
(43) Date of publication of application: **05.04.2023**
(21) Application number: 22190101.0
(22) Date of filing: 18.12.2019
(51) Int. Cl.: C12N 15/70, C12N 15/77, C12N 15/67, C12P 13/04, C07K 14/245

(54) **E. COLI VARIANT STRAIN OR CORYNEBACTERIUM GLUTAMICUM VARIANT STRAIN PRODUCING L-AMINO ACIDS, AND METHOD FOR PRODUCING L-AMINO ACIDS USING SAME**

(30) Priority: 26.12.2018 KR 20180169847
(62) Divisional of application: 19905042.8
(71) Applicant: Daesang Corporation, Seoul 02586 (KR)
(72) Inventor: LEE, Sun Hee, 16923 YONGIN (KR); KIM, Hyun Ho, 05236 SEOUL (KR); KIM, Hyun Young, 16867 YONGIN (KR); JO, Young Il, 04995 SEOUL (KR); KIM, Yong Soo, 16694 SUWON (KR); YANG, Cheol Min, 10474 GOYANG (KR); SHIN, Won Joo, 21318 INCHEON (KR)
(74) Representative: Cabinet Nony

(57) **Abstract**

The present disclosure relates to an L-amino acid-producing *E. coli* mutant strain or *Corynebacterium glutamicum* mutant strain having enhanced L-amino acid productivity, and a method of producing L-amino acid using the same. The L-amino acid-producing *E*. *coli* mutant strain and *Corynebacterium glutamicum* mutant strain according to the present disclosure exhibit an enhanced ability to produce L-amino acid, such as L-tryptophan, L-phenylalanine, L-tyrosine, L-glutamine, L-lysine, L-arginine, L-valine, L-leucine, L-isoleucine, L-threonine, L-histidine, L-serine, or L-citrulline, compared to parent strains thereof, and are capable of producing a high concentration of L-amino acid in high yield.

## Description

### TECHNICAL FIELD

The present disclosure relates to an *E. coli* mutant strain or *Corynebacterium glutamicum* mutant strain having enhanced L-amino acid productivity and a method of producing L-amino acid using the same.

### BACKGROUND ART

L-tryptophan is one of the essential amino acids, and is required to polymerize proteins or synthesize vitamins such as nicotinic acid amide *in vivo,* and is widely used as an amino acid fortifying agent, a feed additive, a raw material for pharmaceuticals such as an infusion solution, and a health food material. L-phenylalanine is one of the essential amino acids, and serves *in vivo* as a precursor of: the non-essential amino acid tyrosine; a neurotransmitter such as dopamine, norepinephrine or adrenaline; or the skin pigment melanin, and is widely used as a raw material for food, cosmetics, and pharmaceuticals. In addition, various amino acids such as L-tyrosine, L-glutamine, L-lysine, L-arginine, L-valine, L-leucine, L-isoleucine, L-threonine, L-histidine, L-serine, and L-citrulline are widely used in various fields such as food, cosmetics and pharmaceuticals.

These L-amino acids were industrially produced by fermentation methods using naturally occurring microbial strains or their mutant strains modified to have enhanced L-amino acid productivity. As the mutant strains, auxotrophic strains or regulatory region mutant strains of microorganisms such as *Escherichia coli* and *Corynebacterium* have been used. Since the 1980s, as genetic recombination technology has rapidly developed and metabolic processes and their regulatory mechanisms have been identified in detail, many researchers have achieved great results by developing excellent recombinant strains using genetic manipulation techniques.

When genetic recombination technology is used, it is possible to enhance amino acid productivity by increasing the activity of an enzyme involved in amino acid biosynthesis or by inhibiting feedback caused by the produced L-amino acid. In addition, it is possible to enhance amino acid productivity by regulating the expression of amino acid exporter genes. US Patent No. 5,972,663 describes artificially overexpressing genes such as *mex, bmr* and *qacA* in several strains (such as *E. coli)* to enhance the abilities of the strains to produce L-cysteine, L-cystine, N-acetylserine and thiazolidine derivatives. European Patent No. 1016710 describes artificially overexpressing the genes *yahN, yeaS, yfiK* and yggA in an *Escherichia* sp. strain to enhance the ability of the strain to produce L-glutamic acid, L-lysine, L-threonine, L-alanine, L-histidine, L-proline, L-arginine, L-valine and L-isoleucine. Korean Patent No. 10-1023925 describes artificially overexpressing the gene *yddG* in an *Escherichia* sp. strain to enhance the ability of the strain to produce L-tryptophan and L-phenylalanine.

Based on these prior studies, the present inventors have constructed L-amino acid-producing mutant strains, which have enhanced ability to produce various L-amino acids, by regulating the expression of L-amino acid exporter genes in an *Escherichia* sp. strain and a *Corynebacterium* sp. strain, thereby completing the present disclosure.

### [Prior Art Documents]

### [Patent Documents]

(Patent Document 1) US Patent No. 5,972,663
(Patent Document 2) European Patent No. 1016710
(Patent Document 3) Korean Patent No. 10-1023925

### DISCLOSURE

### Technical Problem

In order to the above-described problems,
an object of the present disclosure is to provide an *Escherichia coli* mutant strain having enhanced L-amino acid productivity due to overexpression of at least one gene selected from the group consisting of *yicL, ydiN, ydhK, aaeB, yeeA, rhtC* and *emrD.*

Another object of the present disclosure is to provide a *Corynebacterium glutamicum* mutant strain having enhanced L-amino acid productivity due to overexpression of at least one gene selected from the group consisting of *yicL, ydiN, ydhK, aaeB, yeeA, rhtC* and *emrD.*

Still another object of the present disclosure is to provide a method for producing L-amino acid, the method comprising steps of: culturing the mutant strain in a medium; and recovering L-amino acid from the mutant strain or the medium.

### Technical Solution

An L-amino acid-producing mutant strain according to one embodiment of the present disclosure may be an *Escherichia coli* mutant strain or *Corynebacterium glutamicum* mutant strain having enhanced L-amino acid productivity due to overexpression of at least one gene selected from the group consisting of *yicL, ydiN, ydhK, aaeB, yeeA, rhtC* and *emrD.*

In the prior art, there was a case in which the production of the aromatic amino acids L-tryptophan and L-phenylalanine was improved by regulating the amino acid exporter gene *yddG* in an *Escherichia* sp. mutant strain so as to be overexpressed. However, the present inventors examined the effect of the gene *yddG* on the production of L-amino acids, and as a result, confirmed that L-tryptophan and L-phenylalanine were still expressed in the strain from which the gene *yddG* was deleted (see Experimental Example 1). Thereby, the present inventors recognized that genes other than the gene *yddG* are involved in the production of amino acids. In addition, the present inventors selected a new amino acid exporter gene and constructed L-amino acid-producing mutant strains having enhanced ability to produce L-amino acids such as L-tryptophan, L-phenylalanine, L-tyrosine, L-glutamine, L-lysine, L-arginine, L-valine, L-leucine, L-isoleucine, L-threonine, L-histidine, L-serine and L-citrulline.

According to one embodiment of the present disclosure, the amino acid exporter gene may be one of genes represented by the nucleotide sequences of SEQ ID NOs: 1 to 7. Specifically, the amino acid exporter gene may be the gene *yicL* represented by the nucleotide sequence of SEQ ID NO: 1, the gene *ydiN* represented by the nucleotide sequence of SEQ ID NO: 2, the gene *ydhK* represented by the nucleotide sequence of SEQ ID NO: 3 or 37, the gene *aaeB* represented by the nucleotide sequence of SEQ ID NO: 4, the gene *yeeA* represented by the nucleotide sequence of SEQ ID NO: 5, the gene *rhtC* represented by the nucleotide sequence of SEQ ID NO: 6, or the gene *emrD* represented by the nucleotide sequence of SEQ ID NO: 7 or 39.

In addition, the amino acid exporter gene may be one of genes encoding the proteins represented by the amino acid sequences of SEQ ID NOs: 8 to 14. Specifically, the amino acid exporter gene may be a *yicL* protein represented by the amino acid sequence of SEQ ID NO: 8, a *ydiN* protein represented by the amino acid sequence of SEQ ID NO: 9, a *ydhK* protein represented by the amino acid sequence of SEQ ID NO: 10 or 38, an *aaeB* protein represented by the amino acid sequence of SEQ ID NO: 11, a *yeeA* protein represented by the amino acid sequence of SEQ ID NO: 12, an *rhtC* protein represented by the amino acid sequence of SEQ ID NO: 13, or an *emrD* protein represented by the amino acid sequence of SEQ ID NO: 14.

According to one embodiment of the present disclosure, the mutant strain may be selected from an *Escherichia* sp. strain or a *Corynebacterium* sp. strain as a parent strain.

The *Escherichia* sp. strain is widely used for the production of several L-amino acids such as L-glutamic acid, L-lysine, L-threonine and L-cysteine, and is particularly known to have high L-tryptophan productivity. The parent strain is preferably *Escherichia coli,* which is easily available and has good convenience.

The *Corynebacterium* sp. strain has the advantage of low production of by-products while having high ability to produce L-amino acids such as L-glutamic acid and L-lysine, and is capable of stably producing amino acids even in a limited environment such as oxygen deficiency or sugar depletion. The parent strain is preferably *Corynebacterium glutamicum* which is mainly used to produce amino acids.

This parent strain may be a wild-type strain obtained in nature conditions or a strain in which the gene of the wild-type strain has been artificially manipulated.

In the present specification, the term "L-amino acid-producing *Escherichia coli* mutant strain" and "L-amino acid-producing *Corynebacterium glutamicum* mutant strain" refer to microorganisms mutated to have the ability to produce L-amino acids at a higher concentration than the parent strains. At this time, mutagenesis of the microorganisms may be performed by various means well known in the art, and one of physical or chemical mutagenesis methods may be used. For example, as chemical mutagens, N-methyl-N'-nitro-N-nitrosoguanidine (NTG), diepoxybutane, ethylmethane sulfonate, a mustard compound, hydrazine and nitrous acid may be used, but the chemical mutagens are not limited to these compounds. In addition, as physical mutagens, ultraviolet and gamma radiations may be used, but the physical mutagens are not limited thereto. Upon mutagenesis, the parent strain is affected by mutagens at a concentration sufficient to leave a surviving population having a certain size. The size may vary depending on the types of mutagenic factors, and depends on the amount of mutation that is induced in a surviving population at a constant killing rate by the mutagens. For example, when NTG is used, the killing rate may be 10% to 50% of the starting population. Mutagenesis by nitrous acid may be 0.01% to 0.1% of the starting population.

According to one embodiment of the present disclosure, the L-amino acids may be selected from the group consisting of L-tryptophan, L-phenylalanine, L-tyrosine, L-glutamine, L-lysine, L-arginine, L-valine, L-leucine, L-isoleucine, L-threonine, L-histidine, L-serine and L-citrulline, but are not limited thereto.

According to one embodiment of the present disclosure, the L-amino acid-producing *Escherichia coli* mutant strain and the L-amino acid-producing *Corynebacterium glutamicum* mutant strain may overexpress the amino acid exporter gene due to either transformation with the amino acid exporter gene or amplification of the number of copies of the amino acid exporter gene under the control of a strong promoter on the chromosome of each of the strains. According to one embodiment of the present disclosure, it is possible to obtain a mutant strain having enhanced L-amino acid productivity due to overexpression of the amino acid exporter gene compared to the parent strain, by inserting at least one of the seven amino acid exporter genes into a bacterial plasmid containing a promoter and introducing the plasmid into a parent strain.

Using this mutant strain, it is possible to produce a high concentration of L-amino acid in high yield. Specifically, a method for producing L-amino acid may comprise steps of: culturing the mutant strain in a medium; and recovering L-amino acid from the mutant strain or the medium.

In the present specification, the term "medium" refers to a medium which is used in culture of the *Escherichia coli* mutant strain and *Corynebacterium glutamicum* mutant strain of the present disclosure and contains carbon sources, nitrogen sources and inorganic salts so that the mutant strains are capable of producing a high yield of L-amino acid, for example, L-tryptophan, L-phenylalanine, L-tyrosine, L-glutamine, L-lysine, L-arginine, L-valine, L-leucine, L-isoleucine, L-threonine, L-histidine, L-serine or L-citrulline.

Examples of the carbon sources that may be used in the medium include, but are not limited to, saccharides and carbohydrates such as glucose, sugar, citrate, fructose, lactose, maltose or molasses; oils and fats, such as soybean oil, sunflower oil, castor oil or coconut oil; fatty acids such as palmitic acid, stearic acid or linoleic acid; glycerol; alcohols such as ethanol; and organic acids such as acetic acid. These substances may be used individually or as a mixture. Preferably, the medium for the *Escherichia coli* mutant strain may contain glucose. In addition, preferably, the medium for the *Corynebacterium glutamicum* mutant strain may contain glucose, molasses, or glucose and molasses. Most preferably, the medium may contain, as carbon sources, molasses in an amount of 5 to 60 parts by weight based on 100 parts by weight of glucose.

Examples of the nitrogen sources that may be used in the medium include, but are not limited to, peptone, meat extract, yeast extract, dried yeast, corn steep liquor, soybean cake, urea, thiourea, ammonium salt, nitrate, and other compounds organic or inorganic nitrogen. In addition, examples of inorganic salts that may be used in the medium include, but are not limited to, compounds containing magnesium, manganese, potassium, calcium, iron, zinc, cobalt, etc.

Meanwhile, examples of phosphorus sources that may be used in the medium include, but are not limited to, potassium dihydrogen phosphate or dipotassium hydrogen phosphate or the corresponding sodium-containing salts. In addition, the culture medium may contain metal salts such as magnesium sulfate or iron sulfate, which are required for growth. In addition, the above-described compounds may be used individually or as a mixture, but are not limited thereto.

Furthermore, in addition to the carbon source, nitrogen source and inorganic salt components, amino acids, vitamins, nucleic acids and compounds related thereto may be additionally added to the medium of the present disclosure. The above-described components may be added to the medium batchwise or in a continuous manner by a suitable method during culturing.

Meanwhile, the pH of the medium may be adjusted by adding a basic compound such as sodium hydroxide, potassium hydroxide or ammonia, or an acidic compound such as phosphoric acid or sulfuric acid in an appropriate manner. In addition, foaming may be suppressed using an anti-foaming agent such as a fatty acid polyglycol ester. To keep the medium in an aerobic condition, oxygen or an oxygen-containing gas (for example, air) may be injected into the medium.

In the present disclosure, the term "culturing" means growing a microorganism under artificially controlled suitable environmental conditions. Culturing of the microorganism of the present disclosure may be performed using the *Escherichia coli* or *Corynebacterium glutamicum* culturing method widely known in the art. Specifically, examples of the culturing method include, but are not limited to, batch culture, continuous culture, and fed-batch culture.

The temperature of the culturing may generally be, but not limited to, 20°C to 45°C. The culturing may be continued until the largest possible amount of an L-amino acid such as L-tryptophan, L-phenylalanine, L-tyrosine, L-glutamine, L-lysine, L-arginine, L-valine, L-leucine, L-isoleucine, L-threonine, L-histidine, L-serine or L-citrulline is produced. The culturing may be generally performed for, but not limited to, 10 to 160 hours. The produced L-amino acid may be released into the culture medium or contained in the mutant strain.

The method for recovering L-amino acid from the mutant strain or the medium in which the mutant strain has been cultured may be performed using various methods widely known in the art, for example, but not limited to, centrifugation, filtration, anion exchange chromatography, crystallization and HPLC.

### Advantageous Effects

The L-amino acid-producing *Escherichia coli* mutant strain or *Corynebacterium glutamicum* mutant strain according to the present disclosure may exhibit enhanced L-amino acid productivity compared to the parent strain, and may produce a high concentration of L-amino acid in high yield.

### Mode for Invention

Hereinafter, the L-amino acid-producing *Escherichia coli* mutant strain or *Corynebacterium glutamicum* mutant strain according to the present disclosure and the method of producing L-amino acid using the same will be described in more detail with reference to examples. However, this description is provided by way of example only to aid the understanding of the present disclosure, and the scope of the present disclosure is not limited by this illustrative description.

### Experimental Example 1-1. Construction of yddG Gene-Deleted Mutant Strains

In order to examine the effect of the *yddG* gene on L-amino acid production, *yddG* gene-deleted mutant strains were constructed.

The *yddG* gene-deleted mutant strains were constructed using an L-tryptophan-producing *E. coli* WOG strain (accession number: KFCC11660P) and an L-phenylalanine-producing *E. coli* MWTR42 strain (accession number: KFCC10780) as parent strains with reference to the experimental method described in the literature (One-step inactivation of chromosomal genes in Escherichia coli K-12 using PCR products, Datsenko KA, Wanner BL., Proc Natl Acad Sci USA, 2000 Jun 6; 97(12):6640-5).

First, to construct DNA fragments for disruption of the *yddG* gene, polymerase chain reaction (PCR) was performed using the chromosome of each parent strain and a pKD13 plasmid as a template and the primers shown in Table 1 below. At this time, the PCR was performed for a total of 30 cycles, each consisting of (i) denaturation at 95°C for 30 sec, (ii) annealing at 58°C for 30 sec, and (iii) extension at 72°C for 2 min. The PCR products were electrophoresed on 0.8% agarose gel, and bands of desired size were obtained. Using the fragments corresponding to the bands, overlap PCR was performed under the above-described PCR conditions, thereby constructing single DNA fragments for disruption of the *yddG* gene. The DNA fragments were transformed into the respective parent strains which were then streaked on kanamycin-containing solid media and cultured at 37°C for 24 hours. The resultant colonies were subjected to PCR under the above-described PCR conditions.

**[Table 1]**

| **Primer** | **Nucleotide sequence (5' → 3')** |
|---|---|
| yddG H1F (SEQ ID NO: 15) | CAATGCCGCTACTGTTGTTCCAGCC |
| yddG H1R (SEQ ID NO: 16) | CAGTGGTGCGTTTTTCTACCGCTAT |
| yddG H2F (SEQ ID NO: 17) | AATAACTGCCGGGTCTACGGCC |
| yddG H2R (SEQ ID NO: 18) | AACGTATTTTCTAAACGAATTTTAAACGGCG TC |
| yddG CF (SEQ ID NO: 19) | CGGAACAGTATGTGCAGGTGTTACGG |
| yddG CR (SEQ ID NO: 20) | AACAAACCAGTTACAACCACCGCAAC |

As a result, it was confirmed that the *yddG* gene was deleted in each colony.

In addition, the mutant strain obtained by deleting the *yddG* gene from the *E. coli* W0G strain was named W1G strain, and the mutant strain obtained by deleting the *yddG* gene from the *E. coli* MWTR42 strain was named MWTR5 strain.

### Experimental Example 1-2. Examination of L-tryptophan and L-phenylalanine Productivities of yddG Gene-Deleted Mutant Strains

The L-tryptophan productivities of the W0G and W1G strains and the L-phenylalanine productivities of the MWTR42 and MWTR5 strains were examined.

Each of the strains was 1 % inoculated into a flask containing 10 ml of each medium having the composition shown in Table 2 below, and was cultured with shaking at 200 rpm at 37°C for 70 hours. Each culture was measured for absorbance at OD₆₁₀ₙₘ, and the amount of L-tryptophan or L-phenylalanine produced was compared between the strains. The results are shown in Table 3 below.

**[Table 2]**

| **Medium for tryptophan production** | | **Medium for phenylalanine production** | |
|---|---|---|---|
| **Component** | **Content** | **Component** | **Content** |
| Glucose | 80.0 g/L | Glucose | 80.0 g/L |
| (NH₄)₂SO₄ | 20.0 g/L | (NH₄)₂SO₄ | 20.0 g/L |
| K₂HPO₄ | 0.8 g/L | K₂HPO₄ | 1.0 g/L |
| K₂SO₄ | 0.4 g/L | KH₂POa | 1.0 g/L |
| MgCl₂ | 0.8 g/L | K₂SO₄ | 0.4 g/L |
| Fumaric acid | 1.0 g/L | MgCl₂ | 1.0 g/L |
| Yeast extract | 1.0 g/L | Fumaric acid | 0.5 g/L |
| (NH₄)₆MO₇O₂₄ | 0.12 ppm | Yeast extract | 1.0 g/L |
| H₃BO₃ | 0.01 ppm | Glutamic acid | 0.5 g/L |
| CuSO₄ | 0.01 ppm | CaCl₂ | 5.00 ppm |
| MnCl₂ | 2.00 ppm | MnCl₂ | 2.00 ppm |
| ZnSO₄ | 0.01 ppm | ZnSO₄ | 1.00 ppm |
| CoCl₂ | 0.10 ppm | CoCl₂ | 0.10 ppm |
| FeCl₂ | 10.00 ppm | FeCl₂ | 10.00 ppm |
| Thiamine_HCl | 20.00 ppm | Thiamine_HCl | 20.00 ppm |
| L-tyrosine | 200.00 ppm | L-tyrosine | 200.00 ppm |
| L-phenylalanine | 300.00 ppm | CaCO₃ | 3% |
| CaCO₃ | 3% | - | - |

**[Table 3]**

| **Strain** | **L-tryptophan (g/L)** | **Strain** | **L-phenylalanine (g/L)** |
|---|---|---|---|
| W0G | 4.21±0.113 | MWTR42 | 21.3±0.115 |
| W1G | 2.53±0.132 | MWTR5 | 11.7±0.141 |

As shown in Table 3 above, it was confirmed that the *yddG-gene* mutant strains (WIG strain and MWTR5 strain) still produced L-tryptophan and L-phenylalanine, respectively. That is, it could be seen that, even if yddG known as an aromatic amino acid exporter gene was deleted, the L-amino acid productivity of each of the mutant strains was retained, indicating that genes involved in amino acid export are present in addition to *yddG.*

### Experimental Example 2-1. Construction of Mutant Strains Containing the Amplified Genes yicL, ydiN, ydhK, aaeB, yeeA, rhtC and emrD, Respectively

*E. coli* mutant strains into which *yicL, ydiN, ydhK, aaeB, yeeA, rhtC* and *emrD* genes have been inserted, respectively, were constructed.

As a parent strain, an L-tryptophan-producing *E. coli* W0G strain (accession number: KFCC 11660P) was used.

First, each of *yicL, ydiN, ydhK, aaeB, yeeA, rhtC* and *emrD* genes was amplified by PCR using the W0G strain as a template and the primers shown in Table 4 below, and then the PCR products were treated with the restriction enzymes *SacI* and *XbaI*, thereby preparing the genes.

As a result of sequencing, it was confirmed that the *yicL* gene has the nucleotide sequence of SEQ ID NO: 1, the *ydiN* gene has the nucleotide sequence of SEQ ID NO: 2, the *ydhK* gene has the nucleotide sequence of SEQ ID NO: 3 or 37, the *aaeB* gene has the nucleotide sequence of SEQ ID NO: 4, the *yeeA* gene has the nucleotide sequence of SEQ ID NO: 5, the *rhtC* gene has the nucleotide sequence of SEQ ID NO: 6, and the *emrD* gene has the nucleotide sequence of SEQ ID NO: 7 or 39.

The vector pTrc99A was digested by treatment with the restriction enzymes *SacI* and *XbaI,* and each of the prepared genes was inserted into the digested vector, thereby constructing the expression vectors pTrc99A::yicL, pTrc99A::ydiN, pTrc99A::ydhK, pTrc99A::aaeB, pTrc99A::yeeA, pTrc99A::rhtC, and pTrc99A::emrD. Each of the expression vectors was transformed into the W0G strain, thereby constructing the mutant strains W0G/pTrc99A::yicL, W0G/pTrc99A::ydiN, W0G/pTrc99A::ydhK, W0G/pTrc99A::aaeB, W0G/pTrc99A::yeeA, W0G/pTrc99A::rhtC, and W0G/pTrc99A::emrD, which contain each of the amplified genes.

**[Table 4]**

| **Primer** | **Nucleotide sequence (5' → 3')** |
|---|---|
| yicL_F (SEQ ID NO: 21) | GCGAGCTCATGGGTTCCACCAGAAAGGG |
| yicL_R (SEQ ID NO: 22) | GCTCTAGATCACTTATGCCGCGCCGGA |
| ydiN_F (SEQ ID NO: 23) | GCGAGCTCATGTCTCAAAATAAGGCTTTCAGCA |
| ydiN_R (SEQ ID NO: 24) | GCTCTAGAGGCCATCAACCCAATCAATT |
| ydhK_F (SEQ ID NO: 25) | GCGAGCTCATGAACGCATCGTCATGGTCCTTGC |
| ydhK_R (SEQ ID NO: 26) | GCTCTAGATCACTTATGCCGCGCCGGA |
| aaeB_F (SEQ ID NO: 27) | GCGAGCTCATGGGTATTTTCTCCATTGCT |
| aaeB_R (SEQ ID NO: 28) | GCTCTAGATTTTGACTTAACTATCGGTca |
| yeeA_F (SEQ ID NO: 29) | GCGAGCTCGTGCGTGCCGATAAGTC |
| yeeA_R (SEQ ID NO: 30) | GCTCTAGATTATTTGCGCAAGGCCCG |
| rhtC_F (SEQ ID NO: 31) | GCGAGCTCATGTTGATGTTATTTCTCACCGT |
| rhtC_R (SEQ ID NO: 32) | gctctagaCTGGCATCACCGCGAAATAA |
| emrD_F (SEQ ID NO: 33) | GCGAGCTCATGAAAAGGCAAAGAAACG |
| emrD_R (SEQ ID NO: 34) | GCTCTAGACGGTGACGTGCGCTTAAAC |

### Experimental Example 2-2. Examination of L-tryptophan Productivities of Mutant Strains Containing the Amplified Genes yicL, ydiN, ydhK, aaeB, veeA, rhtC and emrD, Respectively

The L-tryptophan productivities of W0G/pTrc99A obtained by inserting only the vector pTrc99A into W0G, and the mutant strains WOG/pTrc99A::yicL, W0G/pTrc99A::ydiN, W0G/pTrc99A::ydhK, W0G/pTrc99A::aaeB, W0G/pTrc99A::yeeA, WOG/pTrc99A::rhtC, and WOG/pTrc99A::emrD, were examined.

Each of the strains was 1 % inoculated into a flask containing 10 ml of each medium having the composition shown in Table 2 above, and was cultured with shaking at 200 rpm at 34°C for 72 hours. Each culture was measured for absorbance at OD_{610nm,} and the amount of L-tryptophan produced was compared between the strains. The results are shown in Table 5 below.

**[Table 5]**

| **Strain** | **L-tryptophan (g/L)** |
|---|---|
| W0G/pTrc99A (control) | 4.21±0.113 |
| WOG/pTrc99A::yicL | 4.83±0.102 |
| W0G/pTrc99 A: :ydiN | 4.86±0.169 |
| W0G/pTrc99 A: :ydhK | 4.88±0.133 |
| W0G/pTrc99 A: :aaeB | 4.91±0.101 |
| WOG/pTrc99A::yeeA | 4.95±0.123 |
| W0G/pTrc99A: :rhtC | 5.02±0.131 |
| WOG/pTrc99A::emrD | 5.26±0.156 |

As shown in Table 5 above, it was confirmed that the L-tryptophan productivities of the *E. coli* mutant strains containing the amplified genes *yicL, ydiN, ydhK, aaeB, yeeA, rhtC* and *emrD*, respectively, increased compared to that of the control.

### Experimental Example 3-1. Construction of Mutant Strains Containing Amplified Genes yicL, ydiN, ydhK, aaeB, yeeA, rhtC and emrD, Respectively

*E. coli* mutant strains into which *yicL, ydiN, ydhK, aaeB, yeeA, rhtC* and *emrD* genes were inserted, respectively, were constructed.

Expression vectors were constructed in the same manner as in Experimental Example 2-1 above, except that an L-phenylalanine-producing *E. coli* MWTR42 strain (accession number: KFCC10780) was used instead of the W0G strain. Each of the expression vectors was transformed into the MWTR42 strain, thereby constructing the mutant strains MWTR42/pTrc99A: :yicL, MWTR42/pTrc99A: :ydiN, MWTR42/pTrc99A: :ydhK, MWTR42/pTrc99A::aaeB, MWTR42/pTrc99A::yeeA, MWTR42/pTrc99A::rhtC, and MWTR42/pTrc99A::emrD, which contain each of the amplified genes.

### Experimental Example 3-2. Examination of L-phenylalanine Productivities of Mutant Strains Containing the Amplified Genes yicL, ydiN, ydhK, aaeB, yeeA, rhtC and emrD, Respectively

The L-phenylalanine productivities of MWTR42/pTrc99A obtained by inserting only the vector pTrc99A into MWTR42, and the mutant strains MWTR42/pTrc99A::yicL, MWTR42/pTrc99A: :ydiN, MWTR42/pTrc99A: :ydhK, MWTR42/pTrc99A: :aaeB, MWTR42/pTrc99A::yeeA, MWTR42/pTrc99A::rhtC, and MWTR42/pTrc99A::emrD, were examined.

The amount of L-phenylalanine produced was compared between the strains in the same manner as in Experimental Example 2-2, and the results are shown in Table 6 below.

**[Table 6]**

| **Strain** | **L-phenylalanine (g/L)** |
|---|---|
| MWTR42/pTrc99A (control) | 21.3±0.115 |
| MWTR42/pTrc99A: :yicL | 27.1±0.081 |
| MWTR42/pTrc99A: :ydiN | 27.2±0.165 |
| MWTR42/pTrc99A: :ydhK | 27.3±0.105 |
| MWTR42/pTrc99A::aaeB | 27.0±0.111 |
| MWTR42/pTrc99A: :yeeA | 27.3±0.103 |
| MWTR42/pTrc99A: :rhtC | 26.7±0.122 |
| MWTR42/pTrc99A::emrD | 27.1±0.085 |

As shown in Table 6 below, it was confirmed that the L-phenylalanine productivities of the *E. coli* mutant strains containing the amplified genes *yicL, ydiN, ydhK, aaeB, yeeA, rhtC* and *emrD*, respectively, increased compared to that of the control.

### Experimental Example 4-1. Construction of Mutant Strains Containing the Amplified Genes yicL, ydiN, ydhK, aaeB, yeeA, rhtC and emrD, Respectively

*Corynebacterium glutamicum* mutant strains into which *yicL, ydiN, ydhK, aaeB, yeeA, rhtC* and *emrD* genes have been inserted, respectively, were constructed.

As a parent strain, L-tryptophan-producing *Corynebacterium glutamicum* DW28G (accession number: KTCT13769BP) was used.

First, each of *yicL, ydiN, ydhK, aaeB, yeeA, rhtC* and *emrD* genes was amplified by PCR using each of the expression vectors (pTrc99A::yicL, pTrc99A::ydiN, pTrc99A::ydhK, pTrc99A::aaeB, pTrc99A::yeeA, pTrc99A::rhtC, and pTrc99A::emrD) constructed in Experimental Examples 2-1 as a template and the primers shown in Table 7 below, and then the PCR products were 5'-phosphorylated, thereby preparing the genes. The vector pEKO was digested by treatment with the restriction enzyme *Eco53KI,* and each of the prepared genes was inserted into the digested vector, thereby constructing the expression vectors pEKO::yicL, pEKO::ydiN, pEKO::ydhK, pEKO::aaeB, pEKO::yeeA, pEKO::rhtC, and pEKO::emrD. Each of the expression vectors was transformed into the DW28G strain, thereby constructing the mutant strains DW28G/pEKO::yicL, DW28G/pEKO::ydiN, DW28G/pEKO::ydhK, DW28G/pEKO::aaeB, DW28G/pEKO::yeeA, DW28G/pEKO::rhtC, and DW28G/pEKO::emrD, which contain each of the amplified genes.

**[Table 7]**

| **Primer** | **Nucleotide sequence (5' → 3')** |
|---|---|
| Primer_F (SEQ ID NO: 35) | gcgccgacatcataacggttctgg |
| Primer_R (SEQ ID NO: 36) | cgcaacgttcaaatccgctcccg |

### Experimental Example 4-2. Examination of L-tryptophan Productivities of Mutant Strains Containing the Amplified Genes yicL, ydiN, ydhK, aaeB, veeA, rhtC and emrD, Respectively

The L-tryptophan productivities of DW28G/pEKO obtained by inserting only the vector pEKO into DW28G, and the mutant strains DW28G/pEKO::yicL, DW28G/pEKO::ydiN, DW28G/pEKO::ydhK, DW28G/pEKO::aaeB, DW28G/pEKO::yeeA, DW28G/pEKO::rhtC, and DW28G/pEKO::emrD, were examined.

The amount of L-tryptophan produced was compared between the strains in the same manner as in Experimental Example 2-2, except that the composition shown in Table 8 was used as a medium. The results are shown in Table 9 below.

**[Table 8]**

| **Component** | **Content** |
|---|---|
| Cane molasses (glucose) | 100.00 g/L |
| KH₂PO₄ | 5.00 g/L |
| K₂HPO₄ | 5.00 g/L |
| K₂SO₄ | 0.40 g/L |
| MgSO₄ | 0.25 g/L |
| (NH₄)₂SO₄ | 20 g/L |
| Corn steep liquor | 10 g/L |
| CaCO₃ | 3% |

**[Table 9]**

| **Strain** | **L-tryptophan (g/L)** |
|---|---|
| DW28G/pEKO (control) | 2.64±0.107 |
| DW28G/pEKO::yicL | 3.57±0.115 |
| DW28G/pEKO::ydiN | 3.05±0.089 |
| DW28G/pEKO::ydhK | 3.11±0.095 |
| DW28G/pEKO::aaeB | 2.95±0.102 |
| DW28G/pEKO::yeeA | 3.21±0.113 |
| DW28G/pEKO: :rhtC | 3.31±0.091 |
| DW28G/pEKO: :emrD | 3.45±0.115 |

As shown in Table 9 above, it was confirmed that the L-tryptophan productivities of the *Corynebacterium glutamicum* mutant strains containing the amplified genes *yicL, ydiN, ydhK, aaeB, yeeA, rhtC* and *emrD*, respectively, increased compared to that of the control.

In conclusion, in the present disclosure, the *E. coli* mutant strain or *Corynebacterium glutamicum* mutant strain having enhanced L-tryptophan or L-phenylalanine productivity compared to the parent strain was obtained by inducing overexpression of the amino acid exporter gene *yicL, ydiN, ydhK, aaeB, yeeA, rhtC* or *emrD*, and it was confirmed that a high concentration of L-tryptophan or L-phenylalanine could be produced in high yield by using this mutant strain.

So far, the present disclosure has been described with reference to the embodiments thereof. Those of ordinary skill in the art to which the present disclosure pertains will appreciate that the present disclosure may be embodied in modified forms without departing from the essential characteristics of the present disclosure. Therefore, the disclosed embodiments should be considered from an illustrative point of view, not from a restrictive point of view. The scope of the present disclosure is defined by the claims rather than the foregoing description, and all differences within the scope equivalent thereto should be construed as being included in the present disclosure.

### EMBODIMENTS OF THE DISCLOSURE

Embodiment 1: A mutant strain having enhanced L-amino acid productivity due to overexpression of at least one gene selected from the group consisting of *yicL, ydiN, ydhK, aaeB, yeeA, rhtC* and *emrD,* the mutant strain being *Escherichia coli* or *Corynebacterium glutamicum.*

Embodiment 2:The mutant strain of claim 1, wherein the *yicL* consists of the nucleotide sequence of SEQ ID NO: 1, the *ydiN* consists of the nucleotide sequence of SEQ ID NO: 2, the *ydhK* consists of the nucleotide sequence of SEQ ID NO: 3 or 37, the *aaeB* consists of the nucleotide sequence of SEQ ID NO: 4, the *yeeA* consists of the nucleotide sequence of SEQ ID NO: 5, the *rhtC* consists of the nucleotide sequence of SEQ ID NO: 6, and the *emrD* consists of the nucleotide sequence of SEQ ID NO: 7 or 39.

Embodiment 3: The mutant strain of claim 1, wherein the L-amino acid is selected from the group consisting of L-tryptophan, L-phenylalanine, L-tyrosine, L-glutamine, L-lysine, L-arginine, L-valine, L-leucine, L-isoleucine, L-threonine, L-histidine, L-serine and L-citrulline.

Embodiment 4: A method for producing L-amino acid, the method comprising steps of:
(a) culturing the mutant strain of claim 1 in a medium; and
(b) recovering L-amino acid from the mutant strain or the medium.

Embodiment 5: The method of claim 4, wherein the L-amino acid is selected from the group consisting of L-tryptophan, L-phenylalanine, L-tyrosine, L-glutamine, L-lysine, L-arginine, L-valine, L-leucine, L-isoleucine, L-threonine, L-histidine, L-serine and L-citrulline.

## Claims

1. A mutant strain having enhanced L-amino acid productivity due to overexpression *of emrD gene,* the mutant strain being *Escherichia coli* or *Corynebacterium glutamicum.*

2. The mutant strain of claim 1, wherein the *emrD* consists of the nucleotide sequence of SEQ ID NO: 7 or 39.

3. The mutant strain of claim 1, wherein the L-amino acid is selected from the group consisting of L-tryptophan, L-phenylalanine, L-tyrosine, L-glutamine, L-lysine, L-arginine, L-valine, L-leucine, L-isoleucine, L-threonine, L-histidine, L-serine and L-citrulline.

4. A method for producing L-amino acid, the method comprising steps of:
(a) culturing the mutant strain of claim 1 in a medium; and
(b) recovering L-amino acid from the mutant strain or the medium.

5. The method of claim 4, wherein the L-amino acid is selected from the group consisting of L-tryptophan, L-phenylalanine, L-tyrosine, L-glutamine, L-lysine, L-arginine, L-valine, L-leucine, L-isoleucine, L-threonine, L-histidine, L-serine and L-citrulline.

6. Use of the mutant strain of claim 1 or 2 for enhancing the production of L-amino acids.

7. The use according to claim 6, wherein the L-amino acid is selected from the group consisting of L-tryptophan, L-phenylalanine, L-tyrosine, L-glutamine, L-lysine, L-arginine, L-valine, L-leucine, L-isoleucine, L-threonine, L-histidine, L-serine and L-citrulline.
